# EUROPEAN PATENT APPLICATION

(11) **EP 4 043 066 A1**
(43) Date of publication of application: **17.08.2022**
(21) Application number: 21156410.9
(22) Date of filing: 10.02.2021
(51) Int. Cl.: A61N 1/32, A61N 1/04

(54) **MACHINE INTERFACE**

(71) Applicant: Nokia Technologies Oy, 02610 Espoo (FI)
(72) Inventor: ZHENG, Mingde, Bridgewater, NJ 08807 (US)
(74) Representative: Swindell & Pearson Limited

(57) **Abstract**

An apparatus comprising: multiple exterior electrodes; and means for controlling input electrical signals provided to a sub-set of the multiple exterior electrodes to control a three-dimensional location in a body of a subject where the input electrical signals combine to selectively stimulate at least first sensational receptors of the body.

## Description

### TECHNOLOGICAL FIELD

Embodiments of the present disclosure relate to a machine interface, for example a human machine interface (HMI).

### BACKGROUND

Using a HMI, it is possible to create electronically a haptic output that physically interacts with a user. An everyday example, is a vibrator in a mobile telephone.

Using a HMI, it is possible create electronically a pixelated light output that represents a high resolution visual image to a user. Everyday examples, include displays for televisions, mobile telephones and computers.

Using a HMI, it is possible create electronically audio output that represents a complex audio scene to a user. Everyday examples, include radios, televisions, mobile telephones and surround sound systems.

A human (and an animal) however have a much wider range of sensations that they can feel, as opposed to see and hear. These sensations include temperature, pain, itch, hair movement, pressure etc.

### BRIEF SUMMARY

It would be desirable to use technology to electronically create such sensations. It would be desirable to selectively stimulate sensational receptors of a body.

According to various, but not necessarily all, embodiments there is provided an apparatus comprising:
multiple exterior electrodes; and
means for controlling input electrical signals provided to a sub-set of the multiple exterior electrodes to control a three-dimensional location in a body of a subject where the input electrical signals combine to selectively stimulate at least first sensational receptors of the body.

In some but not necessarily all examples, combination of the input electrical signals at the three-dimensional location is sufficient to stimulate the first sensational receptors within the three-dimensional location and insufficient to stimulate first sensational receptors outside the three-dimensional location.

In some but not necessarily all examples, the input electrical signals combine, at the three-dimensional location, to exceed a first stimulation threshold associated with the first sensational receptors and to not exceed the first stimulation threshold at locations adjacent the three-dimensional location.

In some but not necessarily all examples, combination of the input electrical signals at the three-dimensional location is sufficient to stimulate the first sensational receptors within the three-dimensional location and insufficient to stimulate at least second sensational receptors within the three-dimensional location, where the first sensational receptors and the second sensational receptors are different.

In some but not necessarily all examples, the input electrical signals combine, at the three-dimensional location, to exceed a first stimulation threshold associated with the first sensational receptors and to not exceed a second stimulation threshold associated with second sensational receptors at the three-dimensional location, where the first sensational receptors and the second sensational receptors are different.

In some but not necessarily all examples, combination of the input electrical signals at the three-dimensional location has a time-varying form and a magnitude engineered to stimulate the first sensational receptors.

In some but not necessarily all examples, the means for controlling the input electrical signals is configured to select which pre-stored time-varying form of input electrical signal is provided to which exterior electrode and at what magnitude.

In some but not necessarily all examples, the means for controlling the input electrical signals is configured to provide a controlled electric current as an input electric signal, wherein the controlled electric current comprises a train of current pulses within an amplitude modulated envelope.

In some but not necessarily all examples, the train of current pulses is additionally frequency modulated.

In some but not necessarily all examples, the envelope is symmetrical and periodic.

In some but not necessarily all examples, the train of current pulses comprises a low frequency low amplitude portion, a high frequency high amplitude portion of relatively shorter duration, a medium frequency medium amplitude portion of relatively longer duration, a high frequency high amplitude portion of relatively shorter duration and a low frequency a low amplitude portion.

In some but not necessarily all examples, the first sensational receptors are selected from the group comprising:
heat receptors,
cold receptors,
pain receptors,
itch sensitive receptors,
hair movement receptors,
mechanoreceptors.

In some but not necessarily all examples, the mechanoreceptors are selected from the group comprising:
Meissner cells,
Merkel cells,
Ruffini cell endings,
Pacinian corpuscles.

In some but not necessarily all examples, the means for controlling input electrical signals provided to a sub-set of the multiple exterior electrodes comprises compensation means for compensating the input electrical signals to account for subject-dependent factors.

In some but not necessarily all examples, the means for controlling input electrical signals provided to a sub-set of the multiple exterior electrodes comprises impedance measurement circuitry configured to estimate variations in impedance between the multiple exterior electrodes and the three-dimensional location.

In some but not necessarily all examples, the apparatus comprises processing means configured to use impedance measurements made by the impedance measurement circuitry to estimate anatomy of the subject and enable matching of the three-dimensional location where the input electrical signals combine to an anatomical location corresponding to first sensational receptors.

In some but not necessarily all examples, the multiple exterior electrodes are integrated into a garment.

According to various, but not necessarily all, embodiments there is provided an apparatus comprising:
means for controlling input electrical signals provided to a sub-set of multiple exterior electrodes to control a three-dimensional location in a subject body where the input electrical signals combine to selectively stimulate at least first sensational receptors of the subject body.

According to various, but not necessarily all, embodiments there is provided a method comprising:
determining input electrical signals provided to a sub-set of multiple exterior electrodes to control a three-dimensional location in a subject body where the input electrical signals combine to selectively stimulate at least first sensational receptors of the subject body; and
providing the determined input electrical signals to the sub-set of multiple exterior electrodes to control the three-dimensional location in the subject body where the input electrical signals combine to selectively stimulate at least first sensational receptors of the subject body.

According to various, but not necessarily all, embodiments there is provided a program that, when run on an apparatus, performs:
controlling input electrical signals provided to a sub-set of multiple exterior electrodes to control a three-dimensional location in a subject body where the input electrical signals combine to selectively stimulate at least first sensational receptors of the subject body.

According to various, but not necessarily all, embodiments there is provided examples as claimed in the appended claims.

### BRIEF DESCRIPTION

Some examples will now be described with reference to the accompanying drawings in which:
FIG. 1 shows an example of an apparatus;
FIG 2 shows an example of electrode;
FIG 3 shows an example of the apparatus in use;
FIG 4A & 4B shows an example of controlling a position of a three-dimensional location in a subject body where the input electrical signals from the electrodes combine to selectively stimulate first sensational receptors in the subject body;
FIG 5 shows an example of selectively stimulating sensational receptors in a subject body;
FIG 6 shows stimulation thresholds of a sensational receptor;
FIG 7 to 14 show examples of input electrical signals
FIG 15 shows examples of anatomical variation of sensational receptors in a subject body;
FIG 16 to 19 illustrates examples of circuitry of the controller;
FIG 20 illustrates an example of a computer program;
FIG 21 illustrates an example of a garment comprising electrodes;
FIG 22 illustrates an example of an operation method.

### DETAILED DESCRIPTION

The following description relates to various examples of an apparatus 10 comprising: multiple exterior electrodes 2; and means 20 for controlling input electrical signals 4 provided to a sub-set 3 of the multiple exterior electrodes 2 to control a three-dimensional location 8 in a subject body 35 where the input electrical signals 4 combine 6 to selectively stimulate at least first sensational receptors 30 in the subject body 35.

The means 20 can be any suitable physical and/or software product. In some, but not necessarily all examples the means 20 is a controller. The controller 20 can, for example, comprise circuitry. The circuitry can be general purpose circuity, that is programmed by firmware or software to perform functions and/or specific purpose circuitry that is designed to perform functions. In the following examples, reference will be made to a controller 20. This should be considered as one of many examples suitable for performing the functions described.

If there are N multiple exterior electrodes 2, and there are M electrodes 2 in the sub-set 3 of electrodes 2, then in at least some examples M is greater than or equal to two, that is the sub-set 3 of electrodes 2 comprises a plurality of electrodes 2. In at least some examples M is less than N. In some examples, M could equal N. In at least some examples, N is a variable that can be controlled and varied by the controller 20.

The electrodes 2 are exterior electrodes in the sense that they are exterior to the body 35 of the subject. The electrodes 2 could alternatively or additionally be called non-invasive electrodes. In this document reference to an electrode 2 is reference to an exterior or non-invasive electrode.

The subject can be a human. In other examples, the subject can be an animal.

No mechanical stimulation of the subject body 35 is required.

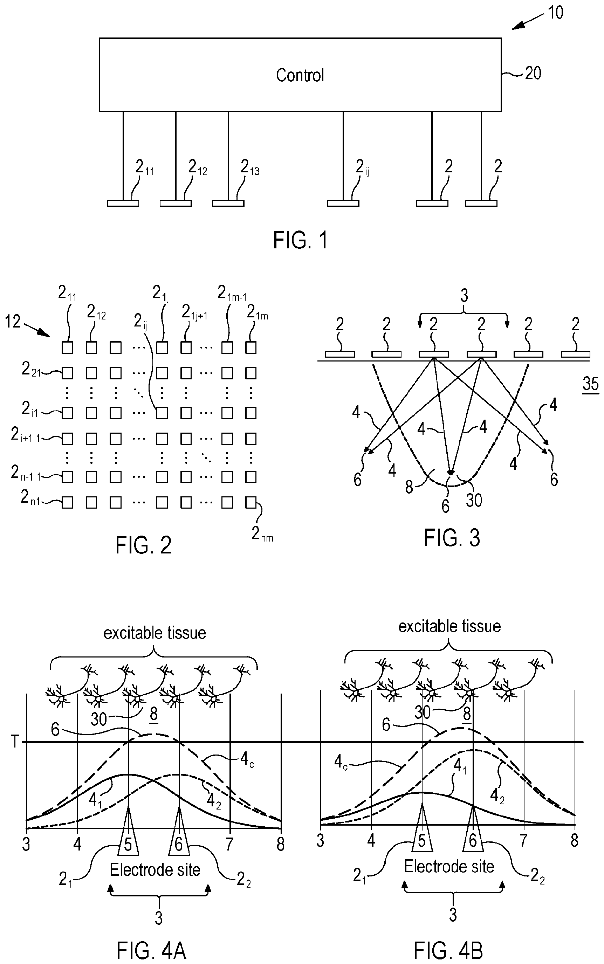

FIG 1 illustrates an example of an apparatus 10 comprising: multiple exterior electrodes 2; and a
controller 20 configured to control input electrical signals 4 provided to a sub-set 3 of the multiple exterior electrodes 2.

The group of electrodes will be referenced using reference 2 (without a subscript). A specific electrode will be reference using reference 2 (with a subscript).

As illustrated in FIG 2 the electrodes 2 can be distributed over a surface area. In this example, the electrodes 2 are arranged in an array 12. In the example illustrated, but not necessarily all examples, the array is a regular array comprising regularly spaced rows and columns of electrodes. In this example, the ray 12 is an array of n rows and m columns. Each row i comprises m distinct electrodes 2ᵢⱼ, where j=1, 2...m, and i=1, 2... n. Each column j comprises n distinct electrodes 2ᵢⱼ, where i=1, 2... n, and j=1, 2...m.

In FIG 3, the exterior electrodes 2 are distributed over a surface area of a body 35 of a subject. The multiple exterior electrodes can, for example, be arranged in an array 12. The controller 20 (not illustrated in this FIG) is configured to control input electrical signals 4 provided to a sub-set 3 of the multiple exterior electrodes 2 to control a three-dimensional location 8 where the input electrical signals 4 combine 6 to selectively stimulate at least first sensational receptors 30 of the body 35.

At the three-dimensional location 8 the input electrical signals 4, from the sub-set 3 of electrodes 2, combine 6 to selectively stimulate sensational receptors 30 of the body 35.

At the three-dimensional location 8 the input electrical signals 4, from the sub-set 3 of electrodes 2, combine 6 and the combination is sufficient to stimulate sensational receptors 30 of the body 35.

At other three-dimensional locations (outside the three-dimensional location 8) the input electrical signals 4, from the sub-set 3 of electrodes 2, combine 6 but the combination is not sufficient to stimulate sensational receptors 30 of the body 35.

The selective stimulation can therefore be selective by location.

In some examples, at the three-dimensional location 8 the input electrical signals 4, from the sub-set 3 of electrodes 2, combine 6 and the combination is sufficient to stimulate a different type of sensational receptor 30 of the body 35. In other examples, at the three-dimensional location 8 the input electrical signals 4, from the sub-set 3 of electrodes 2, combine 6 and the combination is insufficient to stimulate the different type of sensational receptor 30 of the body 35.

The selective stimulation can therefore be selective by type of sensational receptor 30.

The three-dimensional location 8 where the input electrical signals 4, from a sub-set 3 of electrodes 2, combine 6 to stimulate sensational receptors 30 of the body 35 can be moved.

The position of the three-dimensional location 8 where the input electrical signals 4, from a sub-set 3 of electrodes 2, combine 6 to stimulate sensational receptors 30 of the body 35 can be controlled by controlling the sub-set 3 of electrodes and/or by controlling the input electrical signals 4.

For example, the number and location of the sub-set 3 of electrodes can be controlled.

For example, a magnitude of input electrical signal 4 provided by a particular electrode 2 of the sub-set can be controlled.

Controlling the magnitude of input electrical signal 4 provided by the electrodes 2 of the sub-set 3 can be used for electrical beam-steering as illustrated in FIGs 4A and 4B.

In FIG 4A, the input electrical signals 4 provided by the two electrodes 2₁, 2₂ of the sub-set 3 of exterior electrodes 2 have equal magnitude. The input electrical signals 4₁, 4₂ combine 6 to form a combined signal 4_{c} that exceeds a threshold T for stimulating sensational receptors 30 at a position equidistant between the two electrodes 2₁, 2₂.

In FIG 4B, the input electrical signals 4 provided by the two electrodes 2₁, 2₂ of the sub-set 3 of exterior electrodes 2 have unequal magnitude. The input electrical signal 4₁ has a smaller magnitude than the input electrical signal 4₂. The larger input electrical signal 4₂ and the smaller input electrical signal 4₁ combine 6 to form a combined signal 4_{c} that exceeds a threshold T for stimulating sensational receptors 30 at a position that is closer to the electrode 2₂ than the electrode 2₁.

By varying the magnitudes of the input electrical signal 4₁ and the input electrical signal 4₂, the location 8 (where the electrical signals 4₁, 4₂ combine 6 to form a combined signal 4_{c} that exceeds a threshold T for stimulating sensational receptors 30) is varied.

The controller 30, by controlling the magnitudes of the input signals 4, controls a three-dimensional location 8 in a subject body 35 where the input electrical signals 4 combine 6 to selectively stimulate at least first sensational receptors 30 in the subject body.

The controller 30 can, for example, be configured to control which electrodes 2 provide the input signals 4 and/or at what magnitude to change the three-dimensional location 8 where the input electrical signals combine 6 to selectively stimulate sensational receptors 30.

The three-dimensional location 8 can comprise a one or more depths below a surface of the skin and an area at the one or more depths.

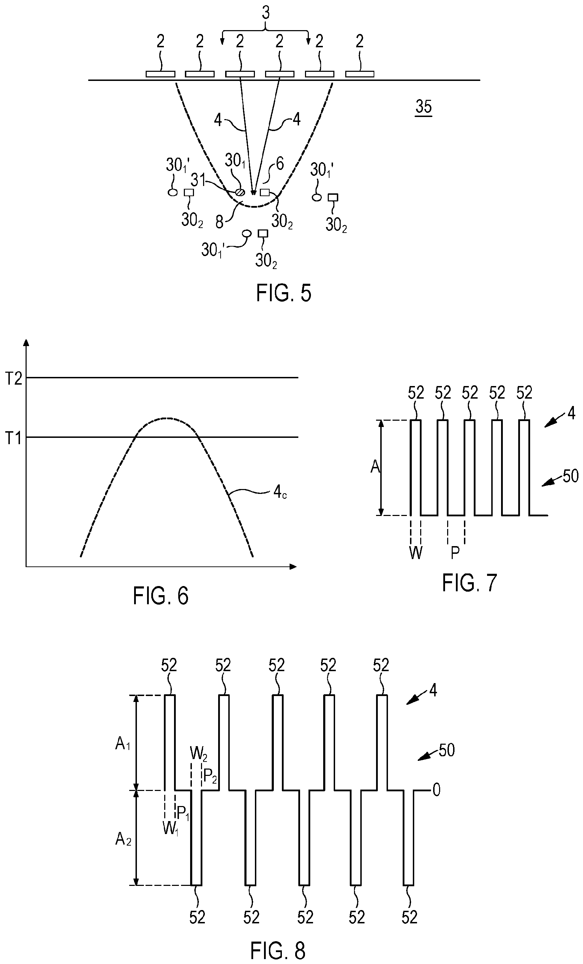

FIG 5 illustrates an example of selectivity of location, where the combination 6 of the input electrical signals 4 at the three-dimensional location 8 is sufficient to stimulate 31 the first sensational receptors 30₁ within the three-dimensional location 8 and is insufficient to stimulate first sensational receptors 30₁' outside the three-dimensional location 8.

FIG 5 also illustrates an example of selectivity of sensational receptor 30, where the combination 6 of the input electrical signals 4 at the three-dimensional location 8 is sufficient to stimulate 31 the first sensational receptors 30₁ within the three-dimensional location 8 and insufficient to stimulate at least second sensational receptors 30₂ within the three-dimensional location 8. The first sensational receptors 30₁ and the second sensational receptors 30₂ are different.

The first sensational receptors 30₁ and the second sensational receptors 30₂ can, for example, be different because they have different histology. The first sensational receptors 30₁ and the second sensational receptors 30₂ can, for example, be different because they induce different sensations at the subject body 35.

FIG 6 schematically illustrates a combination of the input electrical signals 4 from the sub-set 3 of electrodes 2. The input electrical signals 4 from the sub-set 3 of electrodes combine 6 to form a combined signal 4_{c}. The combined signal 4_{c} exceeds a threshold T1 for stimulating first sensational receptors 30₁ only within the selected three-dimensional location 8 and does not exceed a threshold T2 for stimulating second sensational receptors 30₂ whether within the selected three-dimensional location 8 or elsewhere.

In this example, but not necessarily all examples, the input electrical signals 4 from the sub-set 3 of electrodes 2 combine 6, at the three-dimensional location 8, to exceed a first stimulation threshold T1 associated with the first sensational receptors 30₁, 30₁' and to not exceed the first stimulation threshold T1 at locations adjacent the three-dimensional location 8. As a consequence, the first sensational receptors 30₁ inside the three-dimensional location 8 are stimulated and the first sensational receptors 30₁' outside the three-dimensional location 8 are not stimulated (see FIG 5).

In this example, but not necessarily all examples, the input electrical signals 4 from the sub-set 3 of electrodes 2 combine 6, at the three-dimensional location 8, to exceed a first stimulation threshold T1 associated with the first sensational receptors 30 and to not exceed a second stimulation threshold T2 associated with second sensational receptors 30₂ at the three-dimensional location 8. As a consequence, the first sensational receptors 30₁ inside the three-dimensional location 8 are stimulated and the second sensational receptors 30₂ inside the three-dimensional location 8 are not stimulated (see FIG 5). The first sensational receptors 30₁ and the second sensational receptors 30₂ are different, for example, as previously described

As illustrated in FIGs 7 to 14, the input electrical signals 4 can have a time-varying form and a magnitude engineered to stimulate sensational receptors 30.

In some examples, the input electrical signals 4 can have a time-varying form and a magnitude such that they combine 6 at the three-dimensional location 8 so that the combined signal 4_{c} has a time-varying form and a magnitude engineered to stimulate the sensational receptors 30 within the three-dimensional location 8.

In some examples, the input electrical signals 4 can have a particular time-varying form and magnitude such that they combine 6 at the three-dimensional location 8 so that the combined signal 4_{c} has a time-varying form and magnitude engineered to stimulate a particular targeted sensational receptor 30ᵢ within the three-dimensional location 8.

In some examples, the combined signal 4_{c} has a time-varying form and magnitude engineered to not stimulate a particular non-targeted sensational receptor 30ⱼ within the three-dimensional location 8.

The particular targeted sensational receptor 30ᵢ within the three-dimensional location 8 is stimulated but sensational receptors 30ᵢ outside the three-dimensional location 8 are not stimulated and different sensational receptors 30ⱼ (j≠i) inside (and outside) the three-dimensional location 8 are not stimulated.

In at least some examples, the controller 20 is configured to:
select which electrodes provide the electrical signals 4;
maintain production of a selected time-varying form of electrical signal 4 at a selected electrode 2; and
control a magnitude of the electrical signal 4 provided at a selected electrode 2.

In at least some examples, a selected time-varying form of electrical signal 4 can be independently maintained at each selected electrode 2. A magnitude of electrical signal 4 can be independently maintained at each selected electrode 2.

In at least some examples, the controller 20 is configured to control the three-dimensional location 8 where the input electrical signals 4 combine to selectively stimulate particular sensational receptors 20.

In at least some examples, the controller 20 is configured to control the magnitudes of the input electrical signals 4 to select the location 8 of stimulation.

In at least some examples, the controller 20 is configured to change the time-varying form of one or more input electrical signals 4 to select a type of sensational receptor 30 for stimulation. In at least some examples, parameters are stored in a database that define different pre-stored time-varying forms of input electrical signals 4 for stimulating different sensational receptors 30. If a particular sensational receptor 30 is to be targeted the parameters associated with the form of input electrical signal 4 that stimulates the targeted sensational receptor 30 is obtained from the database and used to produce a time-varying form of input electrical signal 4 for stimulating the targeted sensational receptor 30.

In at least some examples, the controller 20 is configured to select which pre-stored time-varying form of input electrical signal 4 is provided to which exterior electrode 2 and at what magnitude.

In at least some examples, the controller 20 is configured to control magnitudes of the input electrical signals 4 and/or forms of the input electrical signals 4 in dependence upon the body 35 of the subject. A calibration process may be used to adapt input electrical signals 4 for use with a particular body 35.

Referring to FIG 7-8 an input electrical signal 4 can comprise a train 50 of pulse 52. For example, a controlled electric current can be used as the input electric signal 4, where the controlled electric current comprises a train 50 of current pulses 52.

FIG 7 illustrates a train 50 of mono-phasic pulses 52. Each pulse is positive only e.g. DC. In this example, each pulse has an amplitude A and a width w. The neighboring pulses 52 are separated by a time period P. In the example illustrated, the amplitude A, the width w and the time period P are constant (fixed). However, in other examples, one or more of the amplitude A, the width w and the time period P vary in time. The train 50 can therefore by amplitude modulated (varying A), pulse-width modulated (varying w) and/or frequency modulated (varying P).

FIG 8 illustrated a train 50 of bi-phasic pulses 52. The train 50 comprises pulses 52 of alternating positive and negative polarity e.g. AC. In this example, each positive polarity pulse has an amplitude A₁ and a width w₁. The positive polarity pulses 52 lag the previous negative polarity pulse 52 by a time period P₁. In this example, each negative polarity pulse has an amplitude A₂ and a width w₂. The negative polarity pulses 52 lag the previous positive polarity pulse 52 by a time period P₂. In the example illustrated, the amplitude A₁, the width w₁ and the time period P₁ are constant (fixed). In the example illustrated, the amplitude A₂, the width w₂ and the time period P₂ are constant (fixed). However, in other examples, one or more of the amplitudes A₁, A₂, the widths w₁, w₂ and the time periods P₁, P₂ vary in time. The train 50 can therefore be amplitude modulated (varying A₁ and/or A₂), pulse-width modulated (varying w₁ and/or w₂) and/or frequency modulated (varying P₁ and/or P₂).

In the particular example illustrated the train 50 of bi-phasic pulses 52 is symmetric in polarity. That is amplitude A₁ equals amplitude A₂, width w₁ equals width w₂ and time period P₁ equals time period P₂. However, in other examples, one or more of the positive polarity parameters (A₁, w₁, P₁) is different or varies differently to the negative polarity parameters (A₂, w₂, P₂).

The form of the pulses 52 can for example be constrained within certain limits.
For example, an amplitude (A, A₁, A₂) can be less than 100mA at lower frequencies and less than 1 mA at higher frequencies. For example, a pulse width (w, w₁, w₂) can be less than 0.5 ms and greater than 1µs. For example, a time period e.g. pitch (P, P₁, P₂) can be between 0.1ms and 20ms.

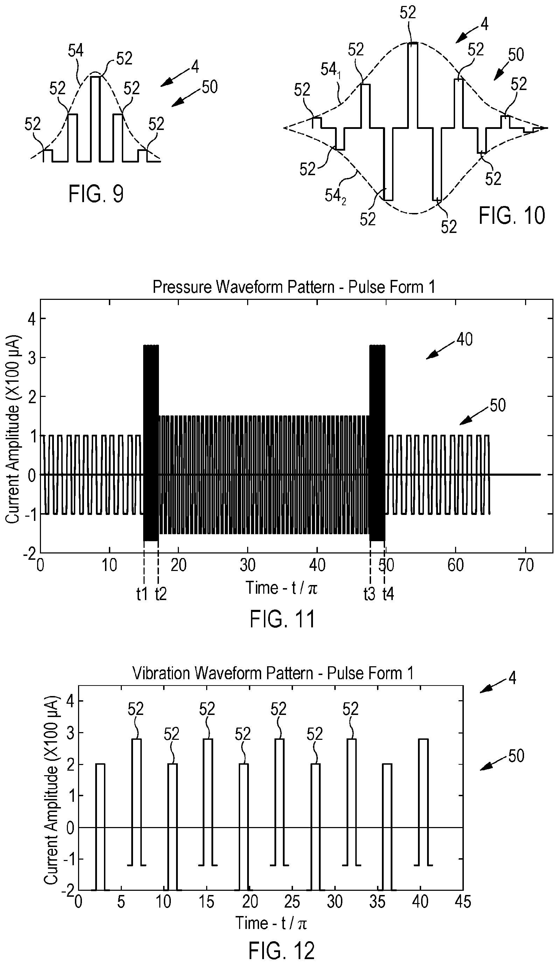

FIG 9 and 10 illustrates a train 50 of pulses 52 that have time-varying amplitudes controlled by time-varying envelopes 54. The description provided for FIGs 8 and 9 is also relevant to FIGs 9 and 10.

The controller 20 is configured to provide a controlled electric current as an input electric signal 4. The controlled electric current comprises a train 50 of current pulses 52 within an amplitude modulated envelope 54.

In some examples, the train of pulses 52 can repeat. In some examples, the repetition is periodic. In other examples, the repetition has a repeat pattern that is repeated for a stimulation. In other examples, the repetition has a pattern that does not repeat for a stimulation

FIG 9 illustrates a train 50 of positive polarity pulses 52 that have time-varying amplitudes controlled by time-varying envelope 54. In this example, the time-varying envelope 54 is symmetric in time. In other examples, the time-varying envelope 54 is asymmetric in time.

FIG 10 illustrates a train 50 of bi-phasic pulses 52 that have time-varying amplitudes controlled by a time-varying envelope 54₁ for the positive polarity pulses 52 and a time-varying envelope 54₂ for the negative polarity pulses 52.

In this example, the time-varying envelope 54₁ is symmetric in time. In other examples, the time-varying envelope 54₁ is asymmetric in time.

In this example, the time-varying envelope 54₂ is symmetric in time. In other examples, the time-varying envelope 54₂ is asymmetric in time.

In this example, the time-varying envelope 54₁ has reflection symmetry with the time-varying envelope 54₂, that is both have the same time-varying form. In other examples, the time-varying envelope 54₁ does not have reflection symmetry with the time-varying envelope 54₂, that is both have different time-varying form.

FIG 11 illustrates an example of a train 50 of bi-phasic pulses 52 that is frequency modulated. It is also amplitude modulated.

The train 50 of pulses 52 comprises a low frequency, low amplitude portion before time t1. The train 50 of pulses 52 comprises a high frequency high amplitude portion between times t1 and t2. The train 50 of pulses 52 comprises a medium frequency medium amplitude portion between times t2 and t3. The train 50 of pulses 52 comprises a high frequency high amplitude portion between times t3 and t4. The train 50 of pulses 52 comprises a low frequency, low amplitude portion after time t4.

The high frequency high amplitude pulses 52 between times t1 and t2 last for a relatively shorter duration (t2-t1) compared to time t3-t2. The medium frequency medium amplitude pulses 52 between times t2 and t3 last for a relatively longer duration (t3-t2) compared to t2-t1. The high frequency high amplitude pulses 52 between times t3 and t4 last for a relatively shorter duration (t4-t3) compared to time t3-t2.

The train 50 of bi-phasic pulses 52 has particular application in stimulating a sensation of pressure.

The low frequency, low amplitude pulses 52 (before time t1, after time t4) can for example have a frequency of the order 50Hz and amplitude of 100µA.

The high frequency high amplitude pulse 52 (between times t1 and t2 and between times t3 and t4) can for example have a frequency of 5 kHz and amplitude of 350µA.

The medium frequency medium amplitude pulses (between times t2 and t3) can for example have a periodicity of the order 500 Hz and amplitude of 150µA.

FIG 12 illustrates an example of a train 50 of bi-phasic pulses 52 that is has a periodic envelope 54₁ ([α₁+A₁cos(2πf₁t)]) for the positive polarity pulses 52 and a time periodic envelope 54₂ (-[α₁+A₂Cos(2πf₂t+ϕ)]) for the negative polarity pulses 52.

In this example, but not necessarily all examples the positive polarity pulses 52 and the negative polarity pulses 52 are time-aligned.

In this example, but not necessarily all examples the mean amplitude of the positive and negative polarity pulses 52 is greater than zero i.e. α₁ > α₂.

In this example, but not necessarily all examples amplitude variation of the positive polarity pulses 52 and the negative polarity pulses is substantially the same i.e. A₁ = A₂.

In this example, but not necessarily all examples a frequency of variation of the positive polarity pulses 52 is the same as a frequency of variation of the negative polarity pulses i.e. f₁ = f₂.

In this example, but not necessarily all examples amplitude variation of the positive polarity pulses 52 is antiphase to variation of the negative polarity pulses i.e. ϕ = π.

In other examples, different fixed values of one or more of α₁, α₂, A₁, A₂, f₁, f₂, ϕ can be used. In other examples, variable values of one or more of α₁, α₂, A₁, A₂, f₁, f₂, ϕ can be used.

The train 50 of bi-phasic pulses 52 has particular application in stimulating a sensation of vibration. Typically f₁, f₂ are in the range 100 Hz- 10kHz.

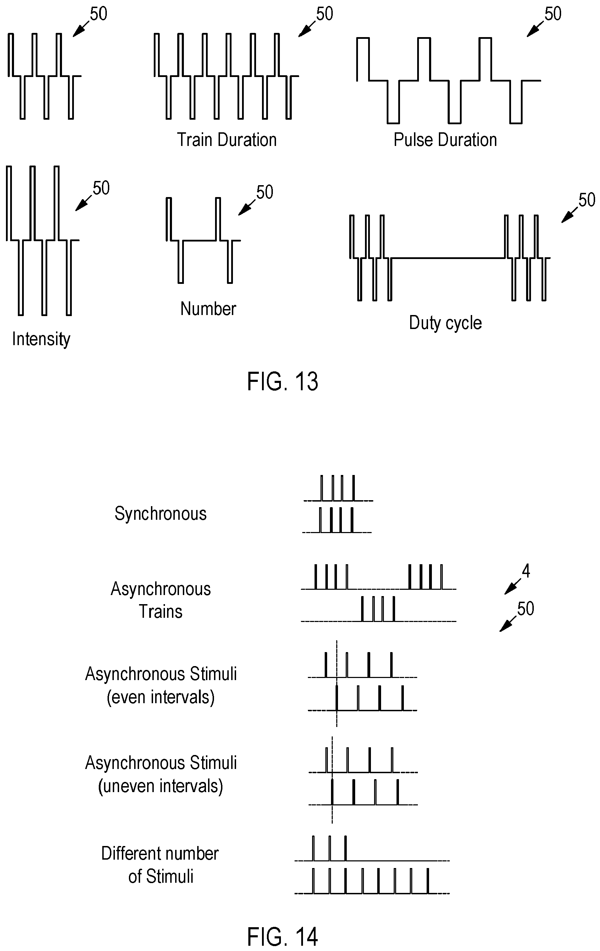

FIG 13 illustrates other examples of inputs signals 5. the input signals 4 comprise trains 50 of pulses 52 (not individually labelled)

The FIG illustrates that different parameters characterizing the train 50 of pulses 52 can be varied including amplitude (intensity), train duration, number of pulses, pulse duration and duty cycle.

FIG 14 illustrates other examples of different inputs signals 4 provided by different electrodes. In this example, the timing of input signal 4 provided by one electrode of the sub-set 3 of electrodes 2 is different from the timing of input signal 4 provided by another electrode of the sub-set 3 of electrodes 2.

In the first example, the trains 50 of pulses provided by the different electrodes 2 of the sub-set 3 are the same, and they are provided synchronously (the pulses are time aligned).

In the second example, the trains 50 of pulses provided by the different electrodes 2 of the sub-set 3 are the same, and they are provided asynchronously. The trains 50 are asynchronous and do not overlap.

In the third and fourth examples, the trains 50 of pulses provided by the different electrodes 2 of the sub-set 3 are the same, and they are provided asynchronously. The trains 50 partially overlap but the pulses of the different trains are not time aligned. In the third example, there are equal intervals between a pulse and the immediately preceding pulse and the immediately following pulse. In the fourth example, there are unequal intervals between a pulse and the immediately preceding pulse and the immediately following pulse.

In the fifth example, the train 50 of pulses provided by one electrode 2 of the sub-set 3 is longer than the train 50 of pulses provided by the other electrode 2 of the sub-set 3. The trains 50 partially overlap and the pulses are time-aligned. The two trains 50 of pulses start synchronously.

The various electrical input electrical signals 4 described, when provided by the sub-set 3 of electrodes 2 combine 6, at the three-dimensional location 8, to mimic biological neural signals at that location 8, for example, by providing synchronous pulse trains or asynchronous pulse trains of the same periodicity or asynchronous pulse trains of different periodicity, where the pulse trains have the same or different lengths.

A human has different sensational receptors 30 and feels different sensations, for example, touch, pressure, vibration, stretch, heat, cold.

The sensational receptors can be targeted by physical (anatomical) location differences within the skin, and also each of these receptors can have its own specific activation passcode/gate that consists of unique amplitude, frequency, and pulse width to initiate spike patterns for the brain to interpret as sensation

The sensational receptors 30 targeted by the controller can, for example, be selected from the group comprising: heat receptors, cold receptors, pain receptors, itch sensitive receptors, hair movement receptors, mechanoreceptors.

Thermoreceptors (sense heat and cold) are located in the dermis layer of the skin and in skeletal muscles. Different stimulatory frequency and amplitude can be used to activate selectively a heat receptor rather than a cold receptor and vice versa. The heat receptor and the cold receptors are stimulated by different pulse train patterns.

Pain receptors (nociceptors) are widely distributed nerve endings in deep tissues (muscles, joints, visceral organs).

Itch sensitive nerves are nerve endings located in the epithelium of the skin.

Hair movement sensors are nerve fibers that wrap around the hair follicles at lower bottom of the dermis layer.

Mechanoreceptors can be selected from the group comprising: Meissner cells, Merkel cells, Ruffini cell endings, Pacinian corpuscles.

Meissner cells can be targeted with a low frequency pulse train (5-50Hz). They are located close to the dermis/epidermis interface.

Merkel cells (disk) can be targeted with a pressure electrical signal 4, for example as illustrated in FIG 11 (0.4-40Hz). They are located close to dermis/epidermis interface.

Ruffini cell endings can be targeted with a lower frequency signal 4 (<7Hz). They are located in the middle dermis.

Pacinian corpuscles can be targeted with a high frequency signal 4 (40-500Hz).

Different receptors 30 can be selectively targeting by selecting the electrodes of the sub-set 3 that are used, the time-varying form of the input signal 4 and the magnitude of the input signal 4.

To a first approximation the magnitude can be used to control 'depth'.

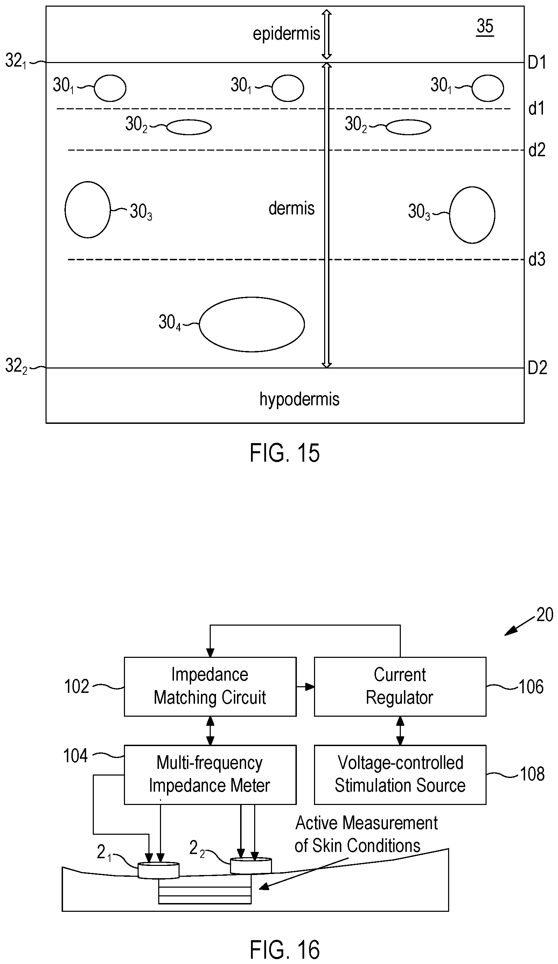

FIG 15 illustrates an example of a body 35. The skin of the body 35 can be considered to be made up of three different layers- the epidermis, the dermis and the hypodermis. The epidermis is nearest the skin surface. the hypodermis is deepest within the skin.

In the example illustrated, the interface 32₁ between the epidermis and the dermis is illustrated and the interface 32₂ between the dermis and hypodermis is illustrated.

The epidermis, dermis and hypodermis can be modelled as different series connected electrical impedances. An electrical signal 4 will travel through the series connected electrical impedances.

By measuring the impedance of the skin of the body, as the skin is probed to increasing depth using an electric signal (probe signal), it is possible to identify where the impedance characteristics change and thus identify a depth of the interfaces 32₁, 32₂.

A knowledge of the depth of the interfaces 32₁, 32₂ enables targeting of specific sensational receptors 30 by targeting a depth relative to the interfaces 32₁, 32₂.

For example, sensational receptors 30₁ can be targeted at a depth between depth D1 of the interface 32₁ and a depth d1. The depth d1 is D1 plus some known proportion k₁ of the distance between the interfaces 32₁, 32₂ (D2-D1) e.g. d1= D1 + ki(D2-D1)

For example, sensational receptors 30₂ can be targeted at a depth between depth d1 and a depth d2. The depth d2 is D1 plus some known proportion k₂ of the distance between the interfaces 32₁, 32₂ (D2-D1) e.g. d2= D1 + k₂(D2-D1).

For example, sensational receptors 30₃ can be targeted at a depth between depth d2 and a depth d3. The depth d3 is D1 plus some known proportion k₃ of the distance between the interfaces 32₁, 32₂ (D2-D1) e.g. d3= D1 + k₃(D2-D1).

For example, sensational receptors 30₃ can be targeted at a depth between depth d3 and the depth D2 of the interface 32₂.

For example, the body can be probed using a probe signal of increasing magnitude which probes an increasing depth. The nth order differential of a measured impedance with distance (voltage) can be determined and used to detect a transition in impedance characteristics indicative of the interface(s) 32₁, 32₂. The impedance characteristic changes abruptly on passing an interface 32₁, 32₂.

The controller 20 can be configured to compensate the input electrical signals 4 provided to stimulate a sensational receptor 30 to account for subject-dependent factors.

One example of a subject dependent factor is a depth of the epidermis or dermis. Other examples of a subject dependent factor are the impedance values of the epidermis, dermis and hypodermis.

Other examples of a subject dependent factor are a contact impedance between the electrodes 2 and the body 35 of the subject.

FIG 16 illustrates an example of compensation circuit that can be used by the controller 20 to compensate the input electrical signals 4 provided to stimulate a sensational receptor 30 to account for subject-dependent factors. This can be described as subject-dependent calibration.

The compensation circuitry comprises impedance measurement circuitry configured to estimate variations in impedance between the multiple exterior electrodes and a three-dimensional location.

The impedance measurement circuitry can also be configured to perform impedance measurements of the subject, with the controller 20 configured to use the impedance measurements to estimate anatomy of the subject and enable matching of the three-dimensional location where the input electrical signals 4 combine to an anatomical location corresponding to first sensational receptors 30.

The compensation circuitry provides a probe current via an electrode 2 on the body of the subject. Pairs of electrodes 2 on the biological specimen measure the voltage as a result of current flow through the subject body with a variable impedance. In some examples, a group of electrodes 2 can be used to guard the electrode 2 used for current injection. The measured voltage is used to recover the impedance information. This can be used to dynamically adjust the input electric signals 4 to respond to a time varying impedance or can be used to assess an anatomy of the subject.

The probe current can be an alternating sine wave of constant amplitude and the voltage measurement can be obtained by demodulated a return current using the probe current as a reference to recover the impedance information.

The various stages are therefore: apply a probe current, measure voltage produced by probe current, determine impedance, and then process the impedance for compensation or other purposes.

In the illustrated example, a multi-frequency impedance meter 104 applies the probe current (this is not a stimulation current) and measures the voltage.

The impedance matching circuit 102 determines impedance.

The current regulator 106 can control a voltage-controlled stimulation source to compensate the input stimulation signals 4 for dynamic variation of impedance or for subject anatomy as previously described.

The multi-frequency impedance meter 104 can have a high output impedance to enable a constant form of current. In some examples, the injected current can be an alternating sine wave of frequency 1 to 100 kHz and with a constant amplitude of 50µA. The multi-frequency impedance meter 104 measures the voltage.

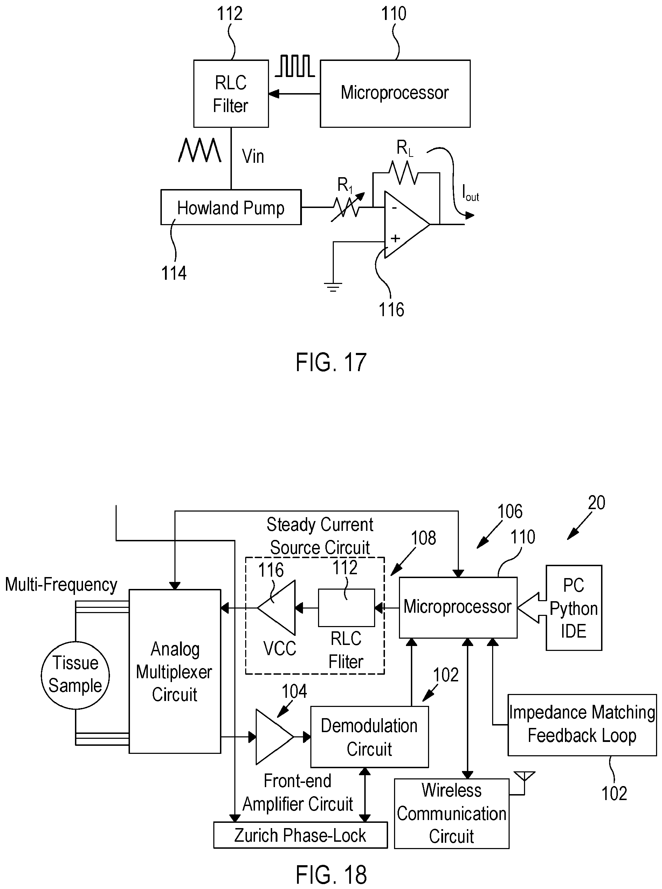

In some examples, the same circuitry can be used, at different times, for generating a probe signal and for generating an input stimulation signal 4. FIG 17 illustrates suitable circuitry. A microprocessor 110 using a direct digital synthesizer generates the desired waveform.

A filtering block 112 can be used to condition the waveforms and suppress any unwanted interferences such as voltage harmonics, and a voltage controlled current source (VCCS) 114, 116 is used to supply a current. A Howland current pump circuit 114 in combination with an operational amplifier 116 is a typical implementation for VCCS.

The microprocessor, 110, filter 112, and voltage controlled current source (VCC) 116 are also illustrated in FIG 18.

FIG 18 illustrates an example of a controller 20.

The voltage-controlled stimulation source 108 can, for example, inject a current, for example as input signal 4 or a probe signal, into the body 35 of the subject.

An analog multiplexer is used for selecting electrodes 2 for use.

The multi-frequency impedance meter 104 measure a voltage in response to a probe signal response provided by amplifier 116. The multi-frequency impedance meter 104 comprises an amplifier and a demodulator. The voltage measurement is demodulated to recover the impedance which is transferred to the microprocessor 110.

The microprocess or 110 operates as current regulator 106 and can control the voltage-controlled stimulation source 108 so that the input signals 4 are compensated for dynamic variation of impedance.

The microprocessor 110 can also process the impedance to assess anatomy of the subject as previously described

The demodulation circuit can be complemented with a phase-lockin amplifier that provides higher accuracy of demodulation at multiple frequencies. The full operation and control of the microprocessor as well as other modules such as the Zurich phase-lockin amplifier are all managed via the microprocessor 110.

A wireless transmission module can be used to communicate the impedance information to remote processing centers.

This compensation system is dynamic, meaning it is constantly sensing, activating, calculating and thresholding at a high sampling rate to beat the variability of skin/contact impedance. At an update rate of 100 Hz, it is more than sufficient to compensate for the biological or environmental variability on the skin surface.

The skin condition is constantly changing for a given individual due to the homeostatic maintenance of the body to regulate waste and temperature, hence the moisture, texture, and contour, anatomy and composition of the skin all would affect the transference of external electrical signal within the skin. Skin condition varies with a person in a given day, as well as with others, hence this variability can be controlled in order to deliver the desired electrical payload resembling sensory stimulation patterns. Depending on the type of electrodes that are in contact with the skin (dry, wet, gel, or non-contact), each electrode introduces an interface that must be electrically compensated for, meaning the degree of contact with the skin needs to be taken into account as to not affect the payload introduced.

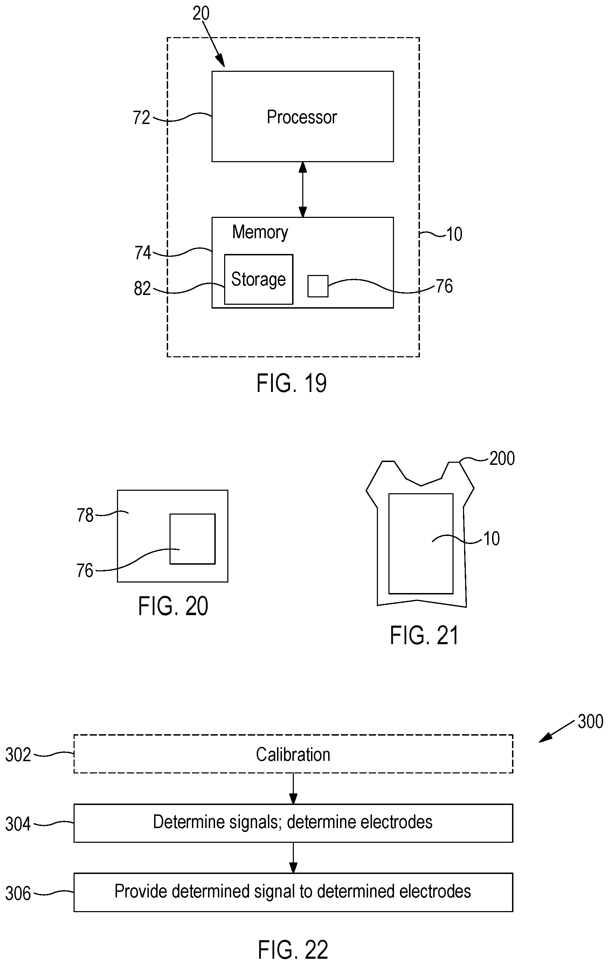

Fig 19 illustrates an example of a controller 20. Implementation of a controller 20 may be as controller circuitry. The controller 20 may be implemented in hardware alone, have certain aspects in software including firmware alone or can be a combination of hardware and software (including firmware).

As illustrated in Fig 19 the controller 20 may be implemented using instructions that enable hardware functionality, for example, by using executable instructions of a computer program 76 in a general-purpose or special-purpose processor 72 that may be stored on a computer readable storage medium (disk, memory etc) to be executed by such a processor 72.

The processor 72 can, in some examples operate as microprocessor 110.

The processor 72 is configured to read from and write to the memory 74. The processor 72 may also comprise an output interface via which data and/or commands are output by the processor 72 and an input interface via which data and/or commands are input to the processor 72.

The memory 74 stores a computer program 76 comprising computer program instructions (computer program code) that controls the operation of the apparatus 10 when loaded into the processor 72. The computer program instructions, of the computer program 76, provide the logic and routines that enables the apparatus to perform the methods illustrated in the Figs. The processor 72 by reading the memory 74 is able to load and execute the computer program 76.

The apparatus 10 therefore comprises:
at least one processor 72; and
at least one memory 74 including computer program code
   the at least one memory 74 and the computer program code configured to, with the at least one processor 72, cause the apparatus 10 at least to perform:
   controlling input electrical signals 4 provided to a sub-set 3 of multiple exterior electrodes 2 to control a three-dimensional location 8 in a subject body 35 where the input electrical signals 4 combine 8 to selectively stimulate at least first sensational receptors 30 of the subject body 35.

In some examples, the apparatus 10 comprises a controller 20 comprising: means for controlling input electrical signals 4 provided to a sub-set 3 of multiple exterior electrodes 2 to control a three-dimensional location 8 in a subject body 35 where the input electrical signals 4 combine 6 to selectively stimulate at least first sensational receptors 30 of the subject body 35.

As illustrated in Fig 20, the computer program 76 may arrive at the apparatus 10 via any suitable delivery mechanism 78. The delivery mechanism 78 may be, for example, a machine readable medium, a computer-readable medium, a non-transitory computer-readable storage medium, a computer program product, a memory device, a record medium such as a Compact Disc Read-Only Memory (CD-ROM) or a Digital Versatile Disc (DVD) or a solid state memory, an article of manufacture that comprises or tangibly embodies the computer program 76. The delivery mechanism may be a signal configured to reliably transfer the computer program 76. The apparatus 10 may propagate or transmit the computer program 76 as a computer data signal.

Computer program instructions for causing an apparatus to perform at least the following or for performing at least the following:
controlling input electrical signals 4 provided to a sub-set 3 of multiple exterior electrodes 2 to control a three-dimensional location 8 in a subject body 35 where the input electrical signals 4 combine to selectively stimulate at least first sensational receptors 30 of the subject body 35.

The computer program instructions may be comprised in a computer program, a non-transitory computer readable medium, a computer program product, a machine readable medium. In some but not necessarily all examples, the computer program instructions may be distributed over more than one computer program.

Although the memory 74 is illustrated as a single component/circuitry it may be implemented as one or more separate components/circuitry some or all of which may be integrated/removable and/or may provide permanent/semi-permanent/ dynamic/cached storage.

Although the processor 72 is illustrated as a single component/circuitry it may be implemented as one or more separate components/circuitry some or all of which may be integrated/removable. The processor 72 may be a single core or multi-core processor.

References to 'computer-readable storage medium', 'computer program product', 'tangibly embodied computer program' etc. or a 'controller', 'computer', 'processor' etc. should be understood to encompass not only computers having different architectures such as single /multi- processor architectures and sequential (Von Neumann)/parallel architectures but also specialized circuits such as field-programmable gate arrays (FPGA), application specific circuits (ASIC), signal processing devices and other processing circuitry. References to computer program, instructions, code etc. should be understood to encompass software for a programmable processor or firmware such as, for example, the programmable content of a hardware device whether instructions for a processor, or configuration settings for a fixed-function device, gate array or programmable logic device etc.

As used in this application, the term 'circuitry' may refer to one or more or all of the following:
(a) hardware-only circuitry implementations (such as implementations in only analog and/or digital circuitry) and
(b) combinations of hardware circuits and software, such as (as applicable):
   (i) a combination of analog and/or digital hardware circuit(s) with software/firmware and
   (ii) any portions of hardware processor(s) with software (including digital signal processor(s)), software, and memory(ies) that work together to cause an apparatus, such as a mobile phone or server, to perform various functions and
(c) hardware circuit(s) and or processor(s), such as a microprocessor(s) or a portion of a microprocessor(s), that requires software (e.g. firmware) for operation, but the software may not be present when it is not needed for operation.
This definition of circuitry applies to all uses of this term in this application, including in any claims. As a further example, as used in this application, the term circuitry also covers an implementation of merely a hardware circuit or processor and its (or their) accompanying software and/or firmware. The term circuitry also covers, for example and if applicable to the particular claim element, a baseband integrated circuit for a mobile device or a similar integrated circuit in a server, a cellular network device, or other computing or network device.

The blocks illustrated in the Figs may represent steps in a method and/or sections of code in the computer program 76. The illustration of a particular order to the blocks does not necessarily imply that there is a required or preferred order for the blocks and the order and arrangement of the block may be varied. Furthermore, it may be possible for some blocks to be omitted.

FIG 21 illustrates an example of an application of the apparatus 10. In this example, at least the external electrodes 2 of the apparatus 10 are external wearable electrodes that are worn on the body of the subject. In the example illustrated, the electrodes 2 are comprised in a garment 200 that can be worn over the body 35 of the subject.

In this example, the garment is shaped as a vest. In other examples, it could be a sleeve for a limb.

Depending on the type of electrodes 2, skin location, and resolution desired, the electrodes can be separated by, for example, between 2mm and 40mm. A higher density of electrodes is desirable at finger tips for example.

The apparatus 10 is also expected to have application for telecommunication, augmented reality, virtual reality, prosthesis, amputees, consumer electronics.

An example of a telecommunication example is the ability to shake hands remotely.

FIG 22 illustrates an example of a method 300.

At block 302, optionally, subject-dependent calibration determines what adaptations are required for the subject body 35. This has been previously described with reference to FIG 16 to 18.

At block 304, the method 300 comprises determining input electrical signals 4 provided to a sub-set 3 of multiple exterior electrodes 2 to control a three-dimensional location 8 in the subject body 35 where the input electrical signals 2 combine 6 to selectively stimulate at least first sensational receptors 30 of the subject body 35.

At block 306, the method 300 comprises providing the determined input electrical signals 4 to the sub-set 3 of multiple exterior electrodes 2 to control the three-dimensional location 8 in the subject body 35 where the input electrical signals 4 combine to selectively stimulate at least first sensational receptors 30 of the subject body 35.

Where a structural feature has been described, it may be replaced by means for performing one or more of the functions of the structural feature whether that function or those functions are explicitly or implicitly described.

In some but not necessarily all examples, the apparatus 10 is configured to communicate data from the apparatus 10 with or without local storage of the data in a memory 74 at the apparatus 10 and with or without local processing of the data by circuitry or processors at the apparatus 10.

The data may, for example, be measured impedance data or data produced by the processing of the measured impedance data, such as, for example, anatomical data.

The data may be stored in processed or unprocessed format remotely at one or more devices. The data may be stored in the Cloud.

The data may be processed remotely at one or more devices. The data may be partially processed locally and partially processed remotely at one or more devices.

The data may be communicated to the remote devices wirelessly via short range radio communications such as Wi-Fi or Bluetooth, for example, or over long range cellular radio links. The apparatus may comprise a communications interface such as, for example, a radio transceiver for communication of data.

The apparatus 10 may be part of the Internet of Things forming part of a larger, distributed network.

The processing of the data, whether local or remote, may be for the purpose of health monitoring, data aggregation, patient monitoring, vital signs monitoring or other purposes.

The processing of the data, whether local or remote, may involve artificial intelligence or machine learning algorithms. The data may, for example, be used as learning input to train a machine learning network or may be used as a query input to a machine learning network, which provides a response. The machine learning network may for example use linear regression, logistic regression, vector support machines or an acyclic machine learning network such as a single or multi hidden layer neural network.

The processing of the data, whether local or remote, may produce an output. The output may be communicated to the apparatus 10 where it may produce an output sensible to the subject such as an audio output, visual output or haptic output.

The systems, apparatus, methods and computer programs may use machine learning which can include statistical learning. Machine learning is a field of computer science that gives computers the ability to learn without being explicitly programmed. The computer learns from experience E with respect to some class of tasks T and performance measure P if its performance at tasks in T, as measured by P, improves with experience E. The computer can often learn from prior training data to make predictions on future data. Machine learning includes wholly or partially supervised learning and wholly or partially unsupervised learning. It may enable discrete outputs (for example classification, clustering) and continuous outputs (for example regression). Machine learning may for example be implemented using different approaches such as cost function minimization, artificial neural networks, support vector machines and Bayesian networks for example. Cost function minimization may, for example, be used in linear and polynomial regression and K-means clustering. Artificial neural networks, for example with one or more hidden layers, model complex relationship between input vectors and output vectors. Support vector machines may be used for supervised learning. A Bayesian network is a directed acyclic graph that represents the conditional independence of a number of random variables.

The above described examples find application as enabling components of: automotive systems; telecommunication systems; electronic systems including consumer electronic products; distributed computing systems; media systems for generating or rendering media content including audio, visual and audio visual content and mixed, mediated, virtual and/or augmented reality; personal systems including personal health systems or personal fitness systems; navigation systems; user interfaces also known as human machine interfaces; networks including cellular, non-cellular, and optical networks; ad-hoc networks; the internet; the internet of things; virtualized networks; and related software and services.

The term 'comprise' is used in this document with an inclusive not an exclusive meaning. That is any reference to X comprising Y indicates that X may comprise only one Y or may comprise more than one Y. If it is intended to use 'comprise' with an exclusive meaning then it will be made clear in the context by referring to "comprising only one.." or by using "consisting".

In this description, reference has been made to various examples. The description of features or functions in relation to an example indicates that those features or functions are present in that example. The use of the term 'example' or 'for example' or 'can' or 'may' in the text denotes, whether explicitly stated or not, that such features or functions are present in at least the described example, whether described as an example or not, and that they can be, but are not necessarily, present in some of or all other examples. Thus 'example', 'for example', 'can' or 'may' refers to a particular instance in a class of examples. A property of the instance can be a property of only that instance or a property of the class or a property of a sub-class of the class that includes some but not all of the instances in the class. It is therefore implicitly disclosed that a feature described with reference to one example but not with reference to another example, can where possible be used in that other example as part of a working combination but does not necessarily have to be used in that other example.

Although examples have been described in the preceding paragraphs with reference to various examples, it should be appreciated that modifications to the examples given can be made without departing from the scope of the claims

Features described in the preceding description may be used in combinations other than the combinations explicitly described above.

Although functions have been described with reference to certain features, those functions may be performable by other features whether described or not.

Although features have been described with reference to certain examples, those features may also be present in other examples whether described or not.

The term 'a' or 'the' is used in this document with an inclusive not an exclusive meaning. That is any reference to X comprising a/the Y indicates that X may comprise only one Y or may comprise more than one Y unless the context clearly indicates the contrary. If it is intended to use 'a' or 'the' with an exclusive meaning then it will be made clear in the context. In some circumstances the use of 'at least one' or 'one or more' may be used to emphasis an inclusive meaning but the absence of these terms should not be taken to infer any exclusive meaning.

The presence of a feature (or combination of features) in a claim is a reference to that feature or (combination of features) itself and also to features that achieve substantially the same technical effect (equivalent features). The equivalent features include, for example, features that are variants and achieve substantially the same result in substantially the same way. The equivalent features include, for example, features that perform substantially the same function, in substantially the same way to achieve substantially the same result.

In this description, reference has been made to various examples using adjectives or adjectival phrases to describe characteristics of the examples. Such a description of a characteristic in relation to an example indicates that the characteristic is present in some examples exactly as described and is present in other examples substantially as described.

Whilst endeavoring in the foregoing specification to draw attention to those features believed to be of importance it should be understood that the Applicant may seek protection via the claims in respect of any patentable feature or combination of features hereinbefore referred to and/or shown in the drawings whether or not emphasis has been placed thereon.

## Claims

1. An apparatus comprising:
multiple exterior electrodes; and
means for controlling input electrical signals provided to a sub-set of the multiple exterior electrodes to control a three-dimensional location in a body of a subject where the input electrical signals combine to selectively stimulate at least first sensational receptors of the body.

2. An apparatus as claimed in claim 1, wherein combination of the input electrical signals at the three-dimensional location is sufficient to stimulate the first sensational receptors within the three-dimensional location and insufficient to stimulate first sensational receptors outside the three-dimensional location.

3. An apparatus as claimed in claim 1 or 2, wherein the input electrical signals combine, at the three-dimensional location, to exceed a first stimulation threshold associated with the first sensational receptors and to not exceed the first stimulation threshold at locations adjacent the three-dimensional location.

4. An apparatus as claimed in any preceding claim, wherein combination of the input electrical signals at the three-dimensional location is sufficient to stimulate the first sensational receptors within the three-dimensional location and insufficient to stimulate at least second sensational receptors within the three-dimensional location, where the first sensational receptors and the second sensational receptors are different form one another.

5. An apparatus as claimed in any preceding claim, wherein the input electrical signals combine, at the three-dimensional location, to exceed a first stimulation threshold associated with the first sensational receptors and to not exceed a second stimulation threshold associated with second sensational receptors at the three-dimensional location, where the first sensational receptors and the second sensational receptors are different from one another.

6. An apparatus as claimed in any preceding claim, wherein combination of the input electrical signals at the three-dimensional location has a time-varying form and a magnitude engineered to stimulate the first sensational receptors.

7. An apparatus as claimed in any preceding claim, wherein the means for controlling the input electrical signals is configured to provide a controlled electric current as an input electric signal, wherein the controlled electric current comprises a train of current pulses within an amplitude modulated envelope.

8. An apparatus as claimed in claim 7, wherein the train of current pulses is additionally frequency modulated.

9. An apparatus as claimed in any preceding claim, wherein the means for controlling input electrical signals provided to a sub-set of the multiple exterior electrodes comprises compensation means for compensating the input electrical signals to account for subject-dependent factors.

10. An apparatus as claimed in any preceding claim, wherein the means for controlling input electrical signals provided to a sub-set of the multiple exterior electrodes comprises impedance measurement circuitry configured to estimate variations in impedance between the multiple exterior electrodes and the three-dimensional location.

11. An apparatus as claimed in claim 10, comprising processing means configured to use impedance measurements made by the impedance measurement circuitry to estimate anatomy of the subject and enable matching of the three-dimensional location where the input electrical signals combine to an anatomical location corresponding to first sensational receptors.

12. An apparatus as claimed in any preceding claim, wherein the multiple exterior electrodes are integrated into a garment.

13. An apparatus comprising:
means for controlling input electrical signals provided to a sub-set of multiple exterior electrodes to control a three-dimensional location in a subject body where the input electrical signals combine to selectively stimulate at least first sensational receptors of the subject body.

14. A method comprising:
determining input electrical signals provided to a sub-set of multiple exterior electrodes to control a three-dimensional location in a subject body where the input electrical signals combine to selectively stimulate at least first sensational receptors of the subject body; and
providing the determined input electrical signals to the sub-set of multiple exterior electrodes to control the three-dimensional location in the subject body where the input electrical signals combine to selectively stimulate at least first sensational receptors of the subject body.

15. A program that, when run on an apparatus, performs:
controlling input electrical signals provided to a sub-set of multiple exterior electrodes to control a three-dimensional location in a subject body where the input electrical signals combine to selectively stimulate at least first sensational receptors of the subject body.
